# EUROPÄISCHE PATENTSCHRIFT

(11) **EP 2 246 074 B1**
(45) Veröffentlichungstag und Bekanntmachung des Hinweises auf die Patenterteilung: **18.02.2015**
(21) Anmeldenummer: 10157201.4
(22) Anmeldetag: 22.03.2010
(51) Int. Cl.: A61L 27/04, B22F 3/10, C22B 26/22, C22C 1/04, C22C 23/00, C22F 1/06

(54) **Verfahren zur Herstellung von Bauteilen aus Magnesium oder Magnesiumlegierung durch Sintern**
Method for producing components from magnesium or magnesium allow with sinters
Procédé de fabrication de composants en magnésium ou en alliage de magnésium par frittage

(30) Priorität: 27.04.2009 DE 102009019041
(43) Veröffentlichungstag der Anmeldung: 03.11.2010
(73) Patentinhaber: Helmholtz-Zentrum Geesthacht Zentrum für Material- und Küstenforschung GmbH, 21502 Geesthacht (DE)
(72) Erfinder: Wolff, Martin, 21481 Lauenburg (DE); Ebel, Thomas, 21447 Handorf (DE); Hort, Norbert, 21339 Lüneburg (DE)
(74) Vertreter: UEXKÜLL & STOLBERG

(56) Entgegenhaltungen:
- US-A- 3 359 095
- US-A1- 2009 081 313

## Beschreibung

Die vorliegende Erfindung betrifft ein Verfahren zur Herstellung von Bauteilen aus Magnesium oder Magnesiumlegierung durch Sintern. Bisher war es nicht möglich Bauteile aus Magnesium oder einer Magnesiumlegierung durch Sintern mit einer brauchbaren Festigkeit, insbesondere einer brauchbaren Druckfestigkeit, und einem brauchbaren Elastizitätsmodul herzustellen.

Solche Bauteile wären beispielsweise als biokompatible und/oder biologisch abbaubare Implantatmaterialien wünschenswert. Bisherige biokompatible Implantatmaterialien wie Stahl- oder Titanlegierungen besitzen den Nachteil eines gegenüber Knochenmaterial deutlich höheren Elastizitätsmoduls und Dichtewertes. Daher kann es zur Lockerung des Implantats kommen. Ein weiteres Problem dieser Implantatmaterialien besteht darin, dass ein selbständiger biologischer Abbau des metallischen Implantats im Körper nicht stattfindet und das Implantat operativ entfernt werden muss.

Zum Zweck der besseren Entfernbarkeit besitzen bekannte metallische Implantate auch glatte Oberflächen. Diese sind jedoch nachteilig, wenn Knochengewebe in das Implantat zur besseren Stabilisierung des Übergangsbereichs einwachsen soll.

Ein Verfahren zur Herstellung von Bauteilen aus einer Magnesiumlegierung, beispielsweise von Implantaten, ist aus US-A-2009/0081313 bekannt.

Bioresorbierbare und/oder biologisch abbaubare, polymere Implantatmaterialien wie solche aus Polylaktiden lassen sich auf Grund ihrer geringen Festigkeit gegenüber metallischen Implantatmaterialien nur in Anwendungsbereichen mit geringer Werkstoffbelastung einsetzen.

Aufgabe der vorliegenden Erfindung ist es daher ein Bauteil zur Verfügung zu Stellen, dass als biokompatibles Implantatmaterial geeignet ist und ein an das Knochenmaterial angepasstes Elastizitätsmodul bei ausreichender Festigkeit aufweisen und dem eine poröse Oberfläche verliehen werden kann sowie ein Verfahren zur Herstellung desselben. Die Implantatmaterialien sollen gleichzeitig auch bioresorbierbar und/oder biologisch abbaubar (biodegradabel) sein.

Ferner ist es Aufgabe der Erfindung, ein Bauteil aus Magnesium oder einer Magnesiumlegierung durch Sintern herzustellen, dass eine brauchbare Festigkeit, wie Druckfestigkeit, und ein brauchbares Elastizitätsmodul ausweist, so dass diese als biokompatible Implantatmaterialien zum Beispiel in Form von Schrauben, Stiften, Bolzen, Haken und/oder Platten geeignet sind.

Gelöst wird das Problem durch ein Verfahren zur Herstellung von Bauteilen aus Magnesium oder Magnesiumlegierungen durch Sintern, bei dem
zunächst ein Grünling aus Magnesiumpulver und/oder einem Magnesiumlegierungspulver und gegebenenfalls einem zusätzlichen Legierungsbestandteil hergestellt wird,
der Grünling in einen inneren Sintertiegel überführt wird,
der innere Sintertiegel in einen äußeren Sintertiegel plaziert wird,
der in dem äußeren Sintertiegel plazierte innere Sintertiegel mit einem Gettermaterial umgeben wird, das in der Lage ist, Gase und/oder Verunreinigungen zu binden,
der äußere Sintertiegel mit dem eingesetzten inneren Sintertiegel und dem Gettermaterial auf Sintertemperatur erwärmt wird und
der äußere Sintertiegel mit dem eingesetzten inneren Sintertiegel und dem Gettermaterial nach erfolgter Sinterung des Grünlings zur Herstellung des Bauteils aus Magnesium oder Magnesiumlegierung abkühlen gelassen wird.

Die Ausgangsmaterialien wie Magnesiumpulver, Magnesiumlegierungspulver und/oder eine Pulvermischung werden vorzugsweise mit einem Druck von 50 bis 125 MPa, und bevorzugter 75 bis 100 MPa gepresst.

Ausschlaggebend für die erfolgreiche Sinterung des Magnesiums oder der Magnesiumlegierung oder auch einer Magnesiumpulvermischung ist die Verwendung eines Gettermaterials. Ein Gettermaterial im Sinne der vorliegenden Erfindung ist ein Material, das in der Lage ist, Gase und/oder Verunreinigungen beim Sinterprozeß zu binden. Bevorzugtes Gettermaterial ist Magnesium, insbesondere Magnesiumpulver.

Vorzugsweise umgibt das Gettermaterial den in dem äußeren Sintertiegel plazierte inneren Sintertiegel. Besonders bevorzugt wird der in dem äußeren Sintertiegel plazierte innere Sintertiegel vollständig von dem Gettermaterial umhüllt. Alternativ kann ein sogenannter "Labyrinth-Sintertiegel-Anordnung" gewählt werden, wobei potentielle Verunreinigungen wie Sauerstoff erst das Gettermaterialbett vollständig passieren müssen, bevor der Innentiegelbereich erreicht wird. Bei einer "Labyrinth-Sintertiegel-Anordnung" können mehrere innere Sintertiegel - durch Zwischenböden getrennt - übereinander angeordnet werden, und werden vorzugsweise von einer Stützhülse abgedeckt und von einer Retorte gehalten.

Der innere und/oder der äußere Sintertiegel kann aus jeglichem Material sein, dass Sintertemperaturen standhalten kann. Besonders bevorzugt sind der innere und/oder der äußere Sintertiegel aus Stahl, vorzugsweise unlegiertem Stahl, gefertigt.

Das Bauteil besteht aus Magnesium oder einer Magnesiumlegierung. Vorzugsweise ist die Magnesiumlegierung eine Magnesium-Calciumlegierung, bevorzugt mit einem Calciumgehalt von bis zu 1,5 Masse%, bevorzugter 0,2 bis 1,0 Masse%, am meisten bevorzugt 0,6 bis 0,8 Masse%. Ein Bauteil aus einer derartigen Magnesium-Calciumlegierung kann beispielsweise durch Verwendung von Magnesium in Kombination mit Calciumhydrid oder Magnesium in Kombination mit einer Magnesium-Calciummasterlegierung als Einsatzmaterialien in entsprechender Menge hergestellt werden. Beispiele zur Herstellung einer Magnesium-Calciumlegierung (MgCa1) unter Verwendung eines Calciumhydrids oder einer Magnesium-Calciummasterlegierung sind wie folgt:

(a) Mg (98,9%) + CaH₂ (1,1%) 〉̶ MgCal (hy)

(b) Mg (98,8%) + MgCa82 (1,2%) 〉̶ MgCal (eu)

(c) Mg (85,5%) + MgCa7 (14,5%) 〉̶ MgCl (al)

Durch Verminderung der Menge an Calciumhydrid oder Magnesium-Calciummasterlegierung werden Magnesium-Calciumlegierungen mit einem Calciumgehalt von weniger als 1 Masse% erhalten.

Bauteile aus einer Magnesium-Calciumlegierung mit einem Calciumgehalt von bis 1,5 Masse%, vorzugsweise bis zu 1 Masse%, am meisten bevorzugt von bis zu 0,8 Masse% sind gegenüber Bauteilen aus reinem Magnesium bevorzugt. Vergleichsversuche haben gezeigt, dass diese Legierungen eine gegenüber reinem Magnesium weiter verbesserte Elastizität und Festigkeit aufweisen.

Als Masterlegierungen zur Herstellung der erfindungsgemäßen Bauteile aus Magnesium-Calciumlegierung können die eutektischen Magnesium-Calciumlegierungen MgCa16 oder MgCa82 oder eine Magnesium-Calciumlegierung mit einem Calciumgehalt von weniger als 16,2 Masse% vorteilhafterweise verwendet werden.

Weiterhin können auch andere Magnesiumlegierungen wie WE43 (4% Yttrium, 3% Seltenerdelemente, Rest Magnesium), oder W4 (4% Yttrium, Rest Magnesium) gemäß dem erfindungsgemäßen Verfahren durch Sinterung hergestellt werden. Vorzugsweise werden auch diese erfindungsgemäßen Legierungen in der Medizintechnik verwendet.

Das Sintern findet vorzugsweise unter Schutzgasatmosphäre oder unter Vakuum statt. Das bevorzugte Schutzgas ist Argon. Bevorzugt findet das Sintern bei Temperaturen unterhalb der Schmelztemperatur von Magnesium statt. Die bevorzugte Sintertemperatur beträgt 600°C bis 642°C. Die Aufheizrate von Umgebungstemperatur zur Sintertemperatur beträgt bevorzugt 0,1 bis 20 K/Minuten, bevorzugter 1 bis 10 K/Minuten, am meisten bevorzugt 3 bis 6 K/Minuten. Die Sinterdauer beträgt vorzugsweise 4 bis 64 Stunden. Eine weitere Verlängerung der Sinterzeit kann die Festigkeitseigenschaften der Proben weiter verbessern. Die Anschließende Abkühlung sollte vorzugsweise möglichst schnell erfolgen, kann aber auch lediglich Abschalten des Ofens erfolgen. Es ist jedoch vorteilhaft, dass das Gettermaterial solange unter Schutzgas oder Vakuum gehalten wird, dass eine Entflammung desselben nicht mehr möglich ist.

Die Erfindung wird durch die nachfolgenden Beispiele erläutert, in denen:
- Fig. 1:: Eine Querschnittsansicht eines äußeren Sintertiegels mit einem darin plazierten inneren Sintertiegel (Nr. 3) und drei in den Sintertiegeln eingebrachten Grünlingen zeigt, wobei kein Gettermaterial vorhanden ist,
- Fig. 2:: Eine Querschnittsansicht eines äußeren Sintertiegels mit zwei darin plazierten inneren Sintertiegeln und jeweils drei in den inneren Sintertiegeln eingebrachten Grünlingen zeigt, wobei ein innerer Sintertiegel (Nr. 1) vollständig mit Gettermaterial umhüllt ist und ein weiterer innerer Sintertiegel (Nr. 2) teilweise von Gettermaterial umgeben ist,
- Fig. 3:: Die Stauchung [%] in Abhängigkeit der Druckfestigkeit [MPa] der drei Proben Nr. 1, Nr. 2 bzw. Nr. 3 zeigt,
- Fig. 4:: Die Stauchung [%] in Abhängigkeit der Druckfestigkeit [MPa] von reinem Magnesium (hergestellt nach einem erfindungsgemäßen Verfahren) bzw. einer MgCa0.6-Legierung (ebenfalls hergestellt nach einem erfindungsgemäßen Verfahren) zeigt.

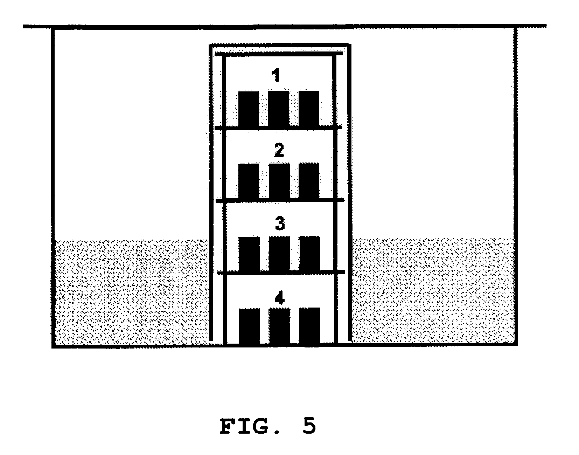

- Fig. 5:: Eine Querschnittansicht einer "Labyrinth-Sintertiegel-Anordnung" ist, wobei mehrere Sintertiegel übereinander angeordnet sind und von einer Stützhülse gehalten und von einer Retorte umgeben sind. Dabei ist die Anordnung so gewählt, dass etwaige Verunreinigungen in Außentiegelbereich erst das Gettermaterial passieren müssen, bevor sie in den Innentiegelbereich gelängen könnten.

### Beispiel 1:

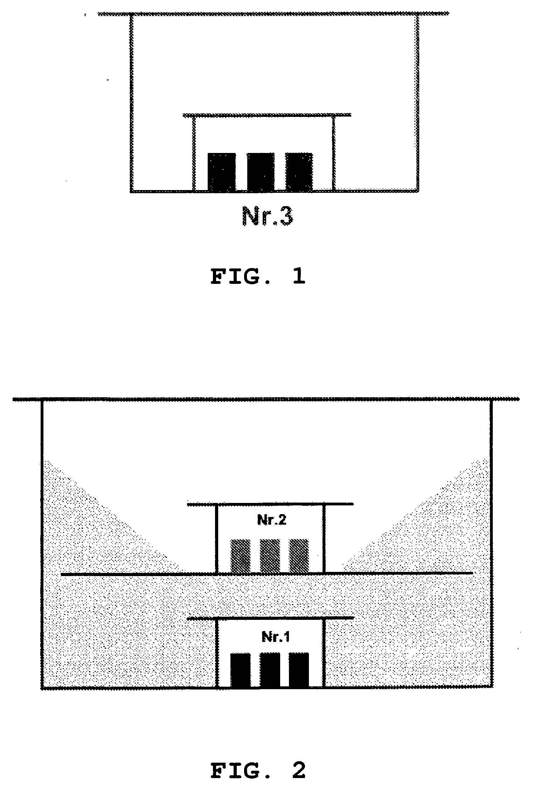

Insgesamt wurden zwölf Grünlinge aus Magnesiumpulver gepresst. Fünf Magnesiumgrünlinge wurden in einem inneren Sintertiegel (Sintertiegel Nr. 1) plaziert, weitere vier Magnesiumgrünlinge wurden in einem inneren Sintertiegel (Sintertiegel Nr. 2) plaziert und schließlich wurden weitere drei Magnesiumgrünlinge in einem inneren Sintertiegel (Sintertiegel Nr. 3) plaziert. Sintertiegel Nr. 3 wurde in einem äußeren Sintertiegel plaziert und nicht mit Gettermaterial umgeben (siehe Figur 1). Sintertiegel Nr. 1 wurde ebenfalls in einem äußeren Sintertiegel plaziert und vollständig mit Magnesiumpulver als Gettermaterial umhüllt, darüber wurde Sintertiegel Nr. 2 plaziert und Magnesiumpulver als Gettermaterial soweit zugegeben, dass Sintertiegel Nr. 2 nur mit Sintermaterial umgeben - nicht vollständig umhüllt - war (siehe Figur 2). Alle Grünlinge wurden in einem Sinterofen unter Argonatmosphäre 64 Stunden bei 630°C gesintert.

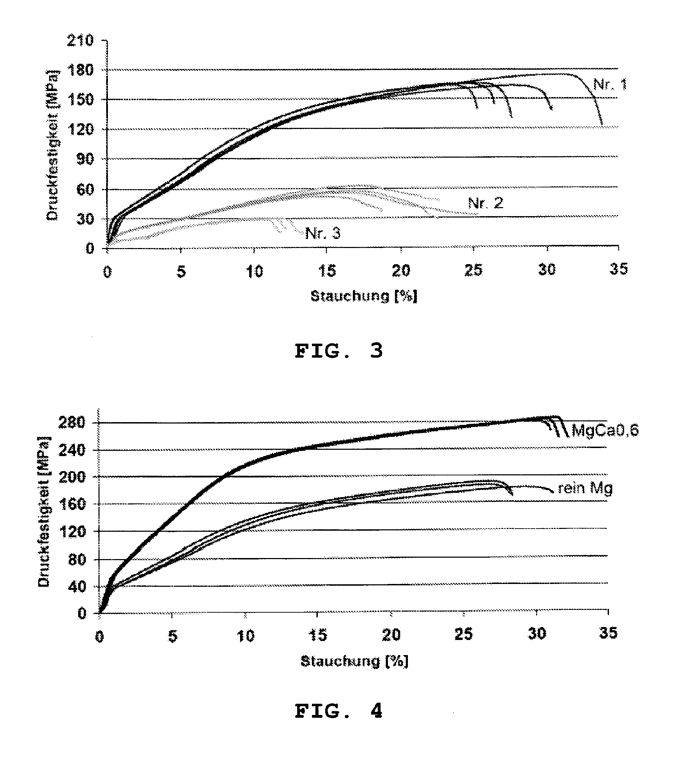

Figur 3 zeigt die Stauchung [%] in Abhängigkeit der Druckfestigkeit [MPa] der drei Proben Nr. 1, Nr. 2 bzw. Nr. 3. Aus Figur 3 ist ersichtlich, dass Probe Nr. 3 (ohne Gettermaterial gesintert) eine sehr geringe Bruchstauchung aufweist. Das Material ist auch wenig elastisch. Andererseits zeigen die Proben, die unter Verwendung eines Gettermaterials gesintert wurden, eine sehr hohe Bruchstauchung und hervorragende Elastizitätswerte, wobei Probe Nr. 1 (mit vollständiger Umhüllung mit Gettermaterial gesintert) die besten Werte aufweist.

| | Druckfestigkeit | 0,2%Dehngrenze | E-Modul | Bruchstauchung | Restporosität |
|---|---|---|---|---|---|
| | [MPa] | [MPa] | [GPa] | [%] | [%] |
| Probe Nr. 1 | 167 ± 4,5 | 35 ± 4 | 8 ± 3 | 26 ± 4 | 14 ± 1 |
| Probe Nr. 2 | 57 ± 4,6 | 18 ± 1 | 2,5 ± 0,5 | 20 ± 3 | 22 ± 1 |
| Probe Nr. 3 | 30 ± 1 | 13,5 ± 6 | 1,2 ± 0,3 | 11 ± 1 | 24 ± 0,3 |

### Beispiel 2:

Es wurden Grünlinge aus Magnesiumpulver sowie Mischungen von Magnesium mit Calciumhydrid bzw. von Magnesium-Caliumlegierungen in folgenden Verhältnissen mit 100 MPa gepresst:

Mg (98,9%) + CaH₂ (1,1%) 〉̶ MgCal (hy)

Mg (98,8%) + MgCa82 (1,2%) 〉̶ MgCal (eu)

Mg (85,5%) + MgCa7 (14,5%) 〉̶ MgCal (al)

Schließlich wurden Grünlinge aus Magnesiumpulver und einer Magnesium-Caliumlegierungen in folgenden Verhältnissen gepresst:

Mg (91,3%) + MgCa7 (8,7%) 〉̶ MgCa0.6 (al)

Die Magnesiumgrünlinge bzw. Magnesiumlegierunggrünlinge wurden in einem inneren Sintertiegel plaziert. Der innere Sintertiegel wurde in einem äußeren Sintertiegel plaziert und vollständig mit Gettermaterial umgeben. Alle Grünlinge wurden in einem Sinterofen unter Argonatmosphäre 64 Stunden bei 630°C gesintert.

**Tabelle 1 zeigt die Werte für die Festigkeit der jeweiligen Magnesium-Calciumlegierungen im Vergleich zu reinem Magnesium und zu MgCa0,6-Gusswerkstoff.**

| | Druckfestigkeit | 0,2%Dehngrenze | E-Modul | Bruchstauchung | Restporosität |
|---|---|---|---|---|---|
| | [MPa] | [MPa] | [GPa] | [%] | [%] |
| reines Mg | 184 ± 7 | 36,2 ± 2,6 | 6,2 ± 2,1 | 26,7 ± 2,7 | 13,6 ± 0,4 |
| MgCa1 (hy) | 217 | 58,4 | 8,8 | 20,6 | 8,0 ± 0,3 |
| MgCa1 (eu) | 236 ± 18,6 | 59,6 | 4,1 | 23,4 | 9,7 ± 0,2 |
| MgCa1 (al) | 255 | 69,4 | 5,9 | 25,5 | 2,1 ± 0,2 |
| MgCa0,6 (al) | 283,7 ± 1,5 | 65,6 ± 4,4 | 8,6 ± 1,6 | 29,6 ± 0,5 | 1,4 ± 0,2 |
| MgCa0,6 (Guss) | 273,2 ± 6,1 | 114,4 ± 15,1 | 44,5 ± 0,8 | - | - |

Figur 4 zeigt die Stauchung [%] in Abhängigkeit der Druckfestigkeit [MPa] von reinem Magnesium und der MgCa0.6-Legierung, die jeweils gemäß Beispiel 2 hergestellt wurden.

## Patentansprüche

1. Verfahren zur Herstellung von Bauteilen aus Magnesium oder Magnesiumlegierung durch Sintern, bei dem
zunächst ein Grünling aus Magnesiumpulver und/oder einem Magnesiumlegierungspulver und gegebenenfalls einem zusätzlichen Legierungsbestandteil hergestellt wird,
der Grünling in einen inneren Sintertiegel überführt wird,
der innere Sintertiegel in einen äußeren Sintertiegel platziert wird,
der in dem äußeren Sintertiegel platzierte innere Sintertiegel mit einem Gettermaterial umgeben wird, das in der Lage ist, Gase und/oder Verunreinigungen zu binden,
der äußere Sintertiegel mit dem eingesetzten inneren Sintertiegel und dem Gettermaterial auf Sintertemperatur erwärmt wird und
der äußere Sintertiegel mit dem eingesetzten inneren Sintertiegel und dem Gettermaterial nach erfolgter Sinterung des Grünlings zur Herstellung des Bauteils aus Magnesium oder Magnesiumlegierung abkühlen gelassen wird.

2. Verfahren nach Anspruch 1, **dadurch gekennzeichnet, dass** der zusätzliche Legierungsbestandteil ausgewählt ist aus der Gruppe bestehend aus Calciumhydrid oder einer Magnesium-Calciummasterlegierung.

3. Verfahren nach Anspruch 2, **dadurch gekennzeichnet, dass** Magnesium-Calciummasterlegierung 2 bis 17 Masse% Calcium enthält.

4. Verfahren nach Anspruch 2, **dadurch gekennzeichnet, dass** Magnesium-Calciummasterlegierung etwa 70 bis 90 Masse% Calcium enthält.

5. Verfahren nach einem der vorgehenden Ansprüche, **dadurch gekennzeichnet, dass** der Grünling unter einer Schutzgasatmosphäre hergestellt wird.

6. Verfahren nach Anspruch 5, **dadurch gekennzeichnet, dass** Argon als Schutzgas verwendet wird.

7. Verfahren nach einem der vorgehenden Ansprüche, **dadurch gekennzeichnet, dass** das Gettermaterial Magnesium ist.

8. Verfahren nach Anspruch 7, **dadurch gekennzeichnet, dass** das Gettermaterial Magnesiumpulver ist.

9. Verfahren nach einem der vorgehenden Ansprüche, **dadurch gekennzeichnet, dass** das Gettermaterial den Sintertiegel vollständig umhüllt.

10. Verfahren nach einem der vorgehenden Ansprüche, **dadurch gekennzeichnet, dass** das die Sintertemperatur 600°C bis 642°C beträgt.

11. Verfahren nach einem der vorgehenden Ansprüche, **dadurch gekennzeichnet, dass** die Sinterdauer 4 bis 64 Stunden beträgt.

12. Verfahren nach einem der vorgehenden Ansprüche, **dadurch gekennzeichnet, dass** die Aufheizrate zur Sinterung 0,1 bis 20 K/min beträgt.

13. Verfahren nach einem der vorgehenden Ansprüche, **dadurch gekennzeichnet, dass** ein Bauteil aus einer Magnesiumlegierung mit 0,1 bis 1% Calcium hergestellt wird.

## Claims

1. Process for producing components consisting of magnesium or magnesium alloy by sintering, in which process
a green compact consisting of magnesium powder and/or a magnesium alloy powder and, if appropriate, an additional alloying constituent is first of all produced,
the green compact is transferred into an inner sintering crucible,
the inner sintering crucible is placed into an outer sintering crucible,
the inner sintering crucible which has been placed in the outer sintering crucible is surrounded with a getter material which is able to bond gases and/or impurities,
the outer sintering crucible with the inner sintering crucible inserted into it and the getter material is heated to sintering temperature, and
the outer sintering crucible with the inner sintering crucible inserted into it and the getter material is allowed to cool after the green compact has been sintered in order to produce the component consisting of magnesium or magnesium alloy.

2. Process according to Claim 1, **characterized in that** the additional alloying constituent is selected from the group consisting of calcium hydride or a magnesium-calcium master alloy.

3. Process according to Claim 2, **characterized in that** the magnesium-calcium master alloy contains 2 to 17% by mass of calcium.

4. Process according to Claim 2, **characterized in that** the magnesium-calcium master alloy contains about 70 to 90% by mass of calcium.

5. Process according to one of the preceding claims, **characterized in that** the green compact is produced in a shielding-gas atmosphere.

6. Process according to Claim 5, **characterized in that** the shielding gas used is argon.

7. Process according to one of the preceding claims, **characterized in that** the getter material is magnesium.

8. Process according to Claim 7, **characterized in that** the getter material is magnesium powder.

9. Process according to one of the preceding claims, **characterized in that** the getter material completely envelops the sintering crucible.

10. Process according to one of the preceding claims, **characterized in that** the sintering temperature is 600°C to 642°C.

11. Process according to one of the preceding claims, **characterized in that** the sintering lasts for 4 to 64 hours.

12. Process according to one of the preceding claims, **characterized in that** the heating rate for sintering is 0.1 to 20 K/min.

13. Process according to one of the preceding claims, **characterized in that** a component is produced from a magnesium alloy containing 0.1 to 1% of calcium.

## Revendications

1. Procédé de fabrication de composants en magnésium ou en alliage de magnésium par frittage, selon lequel
on réalise d'abord une ébauche crue à partir de poudre de magnésium et/ou d'une poudre d'alliage de magnésium et, le cas échéant, d'un composant d'alliage supplémentaire,
on transfère l'ébauche crue dans un creuset de frittage intérieur,
on place le creuset de frittage intérieur dans un creuset de frittage extérieur,
le creuset de frittage intérieur placé dans le creuset de frittage extérieur est entouré d'un matériau dégazeur qui est capable de lier des gaz et/ou des impuretés,
le creuset de frittage extérieur, avec le creuset de frittage intérieur placé dans celui-ci et avec le matériau dégazeur, est chauffé jusqu'à la température de frittage, et
après avoir réalisé le frittage de l'ébauche crue, on laisse refroidir le creuset de frittage extérieur, avec le creuset de frittage intérieur placé dans celui-ci et avec le matériau dégazeur, en vue de la fabrication du composant.

2. Procédé selon la revendication 1, **caractérisé en ce que** le composant d'alliage supplémentaire est choisi dans le groupe comprenant l'hydrure de calcium ou un alliage mère de magnésium-calcium.

3. Procédé selon la revendication 2, **caractérisé en ce que** l'alliage mère de magnésium-calcium contient de 2 à 17 % en masse de calcium.

4. Procédé selon la revendication 2, **caractérisé en ce que** l'alliage mère de magnésium-calcium contient environ de 70 à 90 % en masse de calcium.

5. Procédé selon l'une des revendications précédentes, **caractérisé en ce que** l'ébauche crue est fabriquée sous atmosphère de gaz protecteur.

6. Procédé selon la revendication 5, **caractérisé en ce que** l'argon est utilisé en tant que gaz protecteur.

7. Procédé selon l'une des revendications précédentes, **caractérisé en ce que** le matériau dégazeur est du magnésium.

8. Procédé selon la revendication 7, **caractérisé en ce que** le matériau dégazeur est de la poudre de magnésium.

9. Procédé selon l'une des revendications précédentes, **caractérisé en ce que** le matériau dégazeur entoure complètement le creuset de frittage.

10. Procédé selon l'une des revendications précédentes, **caractérisé en ce que** la température de frittage est comprise entre 600 °C et 642 °C.

11. Procédé selon l'une des revendications précédentes, **caractérisé en ce que** la durée de frittage est comprise entre 4 et 64 heures.

12. Procédé selon l'une des revendications précédentes, **caractérisé en ce que** la vitesse de montée en température pour le frittage est comprise entre 0,1 et 20 K/min.

13. Procédé selon l'une des revendications précédentes, **caractérisé en ce qu'**un composant est fabriqué à partir d'un alliage de magnésium comportant de 0,1 à 1 % de calcium.
